# EUROPÄISCHE PATENTSCHRIFT

(11) **EP 1 967 506 B1**
(45) Veröffentlichungstag und Bekanntmachung des Hinweises auf die Patenterteilung: **26.12.2012**
(21) Anmeldenummer: 08100255.2
(22) Anmeldetag: 09.01.2008
(51) Int. Cl.: C07C 45/78, C07C 45/86, C07C 49/39

(54) **Verfahren zur Stabilisierung von Cyclobutanonderivaten**
Method for stabilising cyclobutanone derivatives
Procédé de stabilisation de dérivés de cyclobutanone

(30) Priorität: 08.03.2007 DE 102007011288
(43) Veröffentlichungstag der Anmeldung: 10.09.2008
(73) Patentinhaber: Evonik Degussa GmbH, 45128 Essen (DE)
(72) Erfinder: Bodmann, Kerstin, 3937, Baltschieder (CH); Imig, Manuela, 63579, Freigericht-Bernbach (DE); Omeis, Marianne, 46286, Dorsten (DE)

(56) Entgegenhaltungen:
- EP-A- 0 764 625
- EP-A- 0 924 180
- WO-A-02/48083

## Beschreibung

Die Erfindung betrifft ein Verfahren zur Stabilisierung von Cyclobutanon als auch ein entsprechend stabilisiertes Cyclobutanon.

Cyclobutanon und seine Derivate sind für einige Herstellungsverfahren von organischen Verbindungen ein wichtiges Edukt. Die Herstellung von Cyclobutanon ist nach unterschiedlichen Verfahren bekannt. So kann Cyclobutanon, beispielweise wie in der DE 101 62 456 beschrieben, durch Umlagerung von Cyclopropylmethanol in wässriger Lösung in Gegenwart eines sauren heterogenen Katalysators und anschließender Dehydrierung unter Verwendung eines heterogenen Katalysators erhalten werden. Ein weiteres Verfahren zur Herstellung von Cyclobutanon beschreibt DE 199 10 464, hierin erfolgt die Herstellung des Cyclobutanons durch Oxidation von Cyclobutanol mittels Alkali- oder Erdalkalihypochlorit.

Die Darstellung von reinem Cyclobutanon stellt eine besondere Herausforderung dar, da das Zielprodukt durch Edukte und durch Nebenkomponenten, die sowohl bei der Herstellung als auch durch die Lagerung des Cyclobutanons entstehen können, in der Regel verunreinigt ist. So beschreibt WO 2002/48083 A2 zahlreiche Verbindungen, die als Verunreinigungen in wässrigem Cyclobutanon nachweisbar sind. Bei diesen Verunreinigungen handelt es sich häufig um farbige Verbindungen, so dass verunreinigtes Cyclobutanon an seiner Farbe zu erkennen ist. So beschreibt die Firma Eastman in der WO 2002/48083 als auch die EP 1 180 509 ein Reinigungsverfahren von rohem Cyclobutanon über fünf Verfahrensstufen, so dass Cyclobutanon in einer Reinheit von > 90% erhalten werden kann.

Trotz diesem aufwändigen Reinigungsverfahren von Cyclobutanon bildet sich nach einigen Tagen oder einigen Monaten ein Niederschlag im Cyclobutanon. Dieser Niederschlag ist für die Verwendung des Cyclobutanons in zahlreichen Herstellungsverfahren sehr störend. Um die Bildung von Verunreinigungen zu reduzieren, wird Cyclobutanon gekühlt und in lichtgeschützten Verpackungen ausgeliefert. Der Mechanismus der Zersetzung des Cyclobutanons wird in einigen Publikationen, wie sie beispielsweise in der WO 2002/48083, aufgelistet sind, beschrieben.

Ein Verfahren zur Verbesserung der Lagerstabilität flüssiger Hydroxy- und Alkoxyalkylketone beschreibt die EP 0 764 625 A1, hierbei werden den acyclischen Ketonen 0,0001 bis 1 Gew.-% einer Base zugesetzt.

Die EP 0 924 180 A1 beschreibt ein Verfahren zur Lagerung von optisch aktiven α-Ketonen, die zur Racemisierung neigen. Das Verfahren wird dadurch ausgezeichnet, dass die Lagerung zum einen in Abwesenheit von Halogenen, Sauerstoff, Metallen, Säuren, Basen und zum anderen in Gegenwart von anorganischen Oxiden erfolgt, welche nicht in der Lage sind Basen oder Säuren auszubilden.

Da Cyclobutanon und seine Derivate als Synthesebaustein für die pharmazeutische Wirkstoffsynthese dient, ist jegliche Verhinderung von Nebenprodukten wünschenswert. Zum einen ist hierbei die Polymerisationstendenz des Cyclobutanons zum anderen die Bildung von Folgeprodukten zu verhindern. So beschreibt die WO 2002/48083 die Zugabe von phenolischen Verbindungen zu Cyclobutanon und seinen Derivaten, die homogen im Produkt verteilt sind, um die Bildung von Nebenprodukten durch Transport und Lagerung zu verhindern, Bei diesen phenolischen Verbindungen handelt es sich um die klassischen Stabilisatoren auf Phenolbasis - sogenannte sterisch gehinderte Phenole, wie beispielsweise 2,6-Di-tert.-butyl-4-methylphenol auch bekannt unter dem Kürzel BHT.

Aufgabe der vorliegenden Erfindung war es nun, ein Verfahren zur Stabilisierung von Cyclobutanon zur Verfügung zu stellen. Insbesondere sollte der zugegebene Stabilisator auch einfach wieder aus dem Cyclobutanon entfernt werden können, damit der Stabilisator selbst nicht störend für die weitere pharmazeutische Wirkstoffsynthese ist. Ferner sollte der verwendete Stabilisator toxisch unbedenklich sein.

Überraschenderweise konnte gefunden werden, dass auch im Cyclobutanon ungelöste Stabilisatoren ausreichend stabilisieren. So führt beispielsweise bereits der Zusatz von 2 Gew.-% Natriumcarbonat oder Calciumoxid zu einer Lagerstabilität des Cyclobutanons über mehrere Wochen. Die vorliegende Erfindung hat gegenüber dem Stand der Technik den Vorteil, dass Natriumcarbonat im Produkt ungelöst vorliegt und somit leicht durch Filtration aus dem Produkt zu entfernen ist. Außerdem handelt es sich bei den in dem erfindungsgemäßen Verfahren eingesetzten Natriumcarbonat um eine toxikologisch unbedenkliche Substanz, die zudem kostengünstig ist.

Gegenstand der Erfindung ist somit ein Verfahren zur Stabilisierung von Cyclobutanon, das sich dadurch auszeichnet, dass dem Cyclobutanon gemäß der Struktur **1** 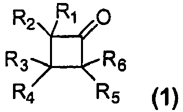 wobei R₁, R₂, R₃, R₄, R₅ und R₆ Wasserstoff, ist Natrium-Carbonat als Stabilisator zugegeben wird.

Weiterer Gegenstand dieser Erfindung ist ein stabilisiertes Cyclobutanon, das sich dadurch auszeichnet, dass das Cyclobutanon gemäß der Struktur 1 Natrium-Carbonatals Stabilisator aufweist.

Das erfindungsgemäße Verfahren zur Stabilisierung von Cyclobutanon zeichnet sich dadurch aus, dass dem Cyclobutanon gemäß der Struktur 1 Natrium-Carbonate als Stabilisator zugegeben wird.

In dem erfindungsgemäßen Verfahren wird Cyclobutanon eingesetzt, das Wasserstoff als Substituenten vom Typ R₁ bis R₆ aufweist.

In dem erfindungsgemäßen Verfahren wird Natriumcarbonat als Stabilisator eingesetzt.

Der Stabilisator- das bedeutet Natrium-Carbonat wird dem Cyclobutanon vorzugsweise in Summe in einer Menge von 0,1 bis 5 Gew.-%, bevorzugt von 0,5 bis 3 Gew.-% bezogen auf das Cyclobutanon in dem erfindungsgemäßen Verfahren zugegeben.

Die Zugabe des Stabilisators erfolgt in dem erfindungsgemäßen Verfahren vorteilhaft als Feststoff. Da sich der in dem erfindungsgemäßen Verfahren eingesetzte Stabilisator nicht in Cyclobutanon löst, ist somit eine einfache Abtrennung des Stabilisators kurz vor dem Einsatz des Cyclobutanon als Edukt in einem Herstellungsverfahren möglich, beispielsweise durch Filtration oder Dekantieren.

Die Zugabe des Stabilisators erfolgt in dem erfindungsgemäßen Verfahren vorzugsweise bei einer Temperatur von 20 bis 30 °C, bevorzugt bei einer Temperatur von 20,5 bis 24 °C und besonders bevorzugt bei Raumtemperatur.

Von Vorteil in dem erfindungsgemäßen Verfahren ist es, den Stabilisator sofort nach der letzten Verfahrenstufe der Aufarbeitung zuzusetzen, damit sich möglichst nicht bereits vor der Zugabe des Stabilisators unerwünschte Nebenprodukte gebildet haben. Bevorzugt erfolgt die Zugabe des Stabilisators unmittelbar nach der Destillation des Cyclobutanon.

Das erfindungsgemäße stabilisierte Cyclobutanon zeichnet sich dadurch aus, dass das Cyclobutanon gemäß der Struktur 1 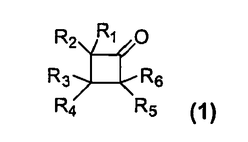 wobei R₁, R₂, R₃, R₄, R₅ und R₆ Wasserstoff, Alkyl-, , ist Natrium-Carbonat als Stabilisator aufweist.

Das erfindungsgemäße stabilisierte Cyclobutanon weist den Stabilisator - in Summe in einer Menge von 0,1 bis 5 Gew.-%, bevorzugt von 0,5 bis 3 Gew.-% bezogen auf das Cyclobutanon auf.

Der Stabilisator liegt in dem erfindungsgemäßen stabilisierten Cyclobutanonüberwiegend als Feststoff vor, insbesondere liegt er als Bodensatz oder auch in Form einer Suspension vor.

Das erfindungsgemäße stabilisierte Cyclobutanon kann gemäß dem erfindungsgemäßen Verfahrens hergestellt werden.

Die nachfolgenden Beispiele sollen das erfindungsgemäße stabilisierte Cyclobutanon als auch seine Herstellung näher erläutern, ohne dass die Erfindung auf diese Ausführungsform beschränkt sein soll.

### Beispiele

5 g Cyclobutanon werden in einem Schnappdeckelgläschen eingewogen und mit jeweils 0,1 g des zu prüfenden Stabilisators versetzt. Die Lagerung erfolgt bei Raumtemperatur. Die Überprüfung der Reinheit erfolgt durch eine Gaschromatographie-Analyse (Säule: 30 m DB-WAX, ID 0,25 mm, FD 0,25 µ; isotherm 50 °C 6 Min. /10 °C/Min. bis 250 °C; die Auswertung erfolgt in Flächenprozent GC-Fl.-%). Die Proben werden vor der Gaschromatographie-Analyse mit Diethylether in einem Verhältnis von Cyclobutanon zu Diethylether von 1 : 2 (bezogen auf das Gewicht) verdünnt.

| Lagerdauer (in Tagen) | ohne Stabilisator (Gehalt an Cyclobutanon in GC-Fl.-%) | CaO (Gehalt an Cyclobutanon in GC-Fl.-%) nicht erfindungsgemäß | Na₂CO₃ (Gehalt an Cyclobutanon in GC-Fl.-%) | K₂CO₃ (Gehalt an Cyclobutanon in GC-Fl.-%) nicht erfindungsgemäß | 2,2,6,6-Tetramethylpiperidin (Gehalt an Cyclobutanon in GC-Fl.-%) * |
|---|---|---|---|---|---|
| 0 | 98,70 | 98,70 | 98,70 | 98,70 | 98,70 |
| 28 | 97,27 | 97,28 | 98,08 | 95,90 | 97,76 |
| 42 | 95,43 | 95,21 | 96,65 | 96,24 | 96,80 |
| 49 | 95,06 | 95,98 | 95,93 | 94,89 | 95,63 |

| | | | | | |
|---|---|---|---|---|---|
| * 2,2,6,6-Tetramethylpiperidin ist ebenfalls ein Stabilisator, scheidet jedoch auf Grund seiner toxikologischen Daten für den Einsatz als Stabilisator für Cyclobutanon in der Praxis aus. | | | | | |

## Patentansprüche

1. Verfahren zur Stabilisierung von Cyclobutanon gemäß der Struktur
**dadurch gekennzeichnet,**
**dass** dem Cyclobutanon gemäß der Struktur 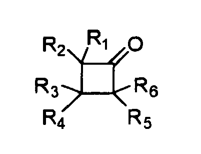 wobei R₁, R₂, R₃, R₄, R₅ und R₆ = Wasserstoff ist,
Natriumcarbonat als Stabilisator zugegeben wird.

2. Verfahren gemäß Anspruch 1,
**dadurch gekennzeichnet,**
**dass** der Stabilisator in einer Menge von 0,1 bis 5 Gew.-%, bezogen auf das Cyclobutanon, dem Cyclobutanon zugegeben wird.

3. Verfahren gemäß Anspruch 1 oder 2,
**dadurch gekennzeichnet,**
**dass** der Stabilisator als Feststoff zugegeben wird.

4. Stabilisiertes Cyclobutanon,
**dadurch gekennzeichnet,**
**dass** das Cyclobutanon gemäß der Struktur 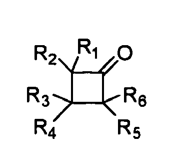 wobei R₁, R₂, R₃, R₄, R₅ und R₆ = Wasserstoff ist,
Natriumcarbonat als Stabilisator aufweist.

5. Stabilisiertes Cyclobutanon gemäß Anspruch 4,
**dadurch gekennzeichnet,**
**dass** das stabilisierte Cyclobutanon den Stabilisator in einer Menge von 0,1 bis 5 Gew.-%, bezogen auf das Cyclobutanon, aufweist.

6. Stabilisiertes Cyclobutanon gemäß Anspruch 4 oder 5,
**dadurch gekennzeichnet,**
**dass** das stabilisierte Cyclobutanon den Stabilisator als Feststoff aufweist.

## Claims

1. Process for stabilizing cyclobutanone,
**characterized in that**
sodium carbonate is added as a stabilizer to the cyclobutanone of the structure 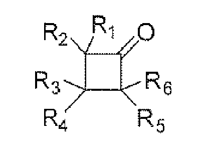 where R₁, R₂, R₃, R₄, R₅ and R₆ are each hydrogen.

2. Process according to Claim 1,
**characterized in that**
the stabilizer is added to the cyclobutanone in an amount of 0.1 to 5% by weight based on the cyclobutanone.

3. Process according to Claim 1 or 2,
**characterized in that**
the stabilizer is added as a solid.

4. Stabilized cyclobutanone,
**characterized in that**
the cyclobutanone of the structure 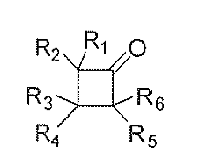 where R₁, R₂, R₃, R₄, R₅ and R₆ are each hydrogen comprises sodium carbonate as a stabilizer.

5. Stabilized cyclobutanone according to Claim 4,
**characterized in that**
the stabilized cyclobutanone comprises the stabilizer in an amount of 0.1 to 5% by weight based on the cyclobutanone.

6. Stabilized cyclobutanone according to Claim 4 or 5,
**characterized in that**
the stabilized cyclobutanone comprises the stabilizer as a solid.

## Revendications

1. Procédé pour la stabilisation de la cyclobutanone,
**caractérisé en ce qu'**on ajoute du carbonate de sodium en tant que stabilisant à la cyclobutanone selon la structure 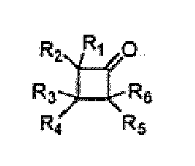 dans laquelle R₁, R₂, R₃, R₄, R₅ et R₆ représentent un atome d'hydrogène.

2. Procédé selon la revendication 1,
**caractérisé en ce qu'**on ajoute à la cyclobutanone le stabilisant en une quantité de 0, 1 à 5 % en poids, par rapport à la cyclobutanone.

3. Procédé selon la revendication 1 ou 2,
**caractérisé en ce qu'**on ajoute le stabilisant sous forme de solide.

4. Cyclobutanone stabilisée,
**caractérisée en ce que** la cyclobutanone selon la structure 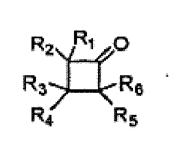 dans laquelle R₁, R₂, R₃, R₄, R₅ et R₆ représentent un atome d'hydrogène,
comporte du carbonate de sodium en tant que stabilisant.

5. Cyclobutanone stabilisée selon la revendication 4,
**caractérisée en ce que** la cyclobutanone stabilisée comporte le stabilisant en une quantité de 0,1 à 5 % en poids, par rapport à la cyclobutanone.

6. Cyclobutanone stabilisée selon la revendication 4 ou 5,
**caractérisée en ce que** la cyclobutanone stabilisée comporte le stabilisant sous forme de solide.
